# EUROPEAN PATENT SPECIFICATION

(11) **EP 1 882 494 B1**
(45) Date of publication and mention of the grant of the patent: **17.11.2010**
(21) Application number: 07013606.4
(22) Date of filing: 11.07.2007
(51) Int. Cl.: A61N 1/44, A61H 33/12

(54) **Skin care appliance**
Hautpflegegerät
Applicateur de soin dermatologique

(30) Priority: 26.07.2006 JP 2006203940
(43) Date of publication of application: 30.01.2008
(73) Proprietor: Panasonic Electric Works Co., Ltd., Kadoma-shi Osaka (JP)
(72) Inventor: Oomura, Shingo, Kadoma-shi Osaka (JP); Fujiwara, Mitsuru, Kadoma-shi Osaka (JP); Kishimoto, Takashi, Kadoma-shi Osaka (JP)
(74) Representative: Appelt, Christian W.

(56) References cited:
- EP-A- 1 607 080

## Description

### TECHNICAL FIELD

The invention relates to skin care appliances and more particularly to a skin care appliance for steam beauty treatment with antibacterial action.

### BACKGROUND ART

Japanese Patent Application Publication No. H10-295432 issued Nov. 10, 1998 discloses a steam generating appliance. This appliance comprises a steam passage through which steam can flow to the outside; an ion passage connected to the intermediate position of the steam passage so that air can flow in the steam passage through the ion passage; and a pair of discharge electrodes located in the ion passage. When ions are generated by the (arc) discharge between the electrodes, the ions flow in the steam passage to become cores for the steam and the steam components condense on the cores to become minute.

Japanese Patent Application Publication No. 2005-349132 issued Dec. 22, 2005 discloses a steam beauty appliance. This appliance comprises a high voltage discharger that is located in a passage through which steam can flow and makes the steam finer.

Japanese Patent Application Publication No. 2003-159305 issued Jun. 3, 2003 discloses a skin care appliance that utilizes steam and minus ions for skin care. This appliance uses corona discharge, self-induced radiation (tourmaline), electrostatic spray or water pulverization to generate minus ions. Since the minus ions have antibacterial action, suitable skin care is possible.

Japanese Patent Application Publication No. 2004-16523 issued Jan. 22, 2004 also discloses a facial appliance that utilizes steam and minus ions for skin care. This appliance effectively increases quantity of minus ions generated by a minus ion generator (high voltage) and thereby improves skin care with the minus ions.

There is a need for an improved skin care appliance that displays more suitable antibacterial action than minus ions generated with corona discharge (high voltage), self-induced radiation (tourmaline), electrostatic spray or water pulverization and can give the antibacterial action more uniformly to human skin.

Furthermore, EP 1 607 080 A1 on which the preambles of claims 1 and 9 are based discloses a steam generator with a liquid reservoir, a boiler for heating the liquid from the liquid reservoir, a nozzle and a discharge generating portion comprising a pair of electrodes.

### DISCLOSURE OF THE INVENTION

It is therefore an object of the present invention to provide more suitable antibacterial action than minus ions generated with corona discharge (high voltage), self-induced radiation (tourmaline), electrostatic spray or water pulverization and give the antibacterial action more uniformly to human skin.

According to an aspect of the present invention, a skin care appliance (hereinafter referred to as a "first appliance") comprises a liquid reservoir, a boiler, a nozzle and a pair of discharge electrodes. The liquid reservoir keeps liquid. The boiler is connected to the liquid reservoir through a feed passage and heats the liquid from the liquid reservoir to generate steam. The nozzle is connected to the boiler through a steam passage and directs the steam from the boiler to the outside. The discharge electrodes include metal with antibacterial action and are located between the boiler and an opening of the nozzle. The electrodes scatter metal ions derived from the metal by electric discharge generated between the electrodes. In addition, components of a substance for separation of the metal ions are supplied between the boiler and the opening of the nozzle, and the components prevent combination among the metal ions.

In this configuration, the metal ions become cores for the steam and the steam components condense on the cores to become minute. As a result, it is possible to provide human skin with steam (metal ions) having more suitable antibacterial action than minus ions generated with corona discharge (high voltage), self-induced radiation (tourmaline), electrostatic spray or water pulverization. Moreover, since the components of the substance for separation of the metal ions prevent combination among the metal ions, the antibacterial action derived from the metal ions can be give to the skin more uniformly.

Preferably, the skin care appliance (hereinafter referred to as a "second appliance") further comprises an intermediate electrode that includes metal with antibacterial action and is located between the discharge electrodes. The intermediate electrode further scatters metal ions derived from the metal included in the intermediate electrode by electric discharge generated between the intermediate electrode and one of the discharge electrodes as well as electric discharge generated between the intermediate electrode and the other of the discharge electrodes. In this case, antibacterial action of the steam (metal ions) can be heightened.

According to an aspect of the present invention, a skin care appliance (hereinafter referred to as a "third appliance")comprises a liquid reservoir, a boiler, a nozzle, a pair of discharge electrodes and an intermediate electrode. The liquid reservoir keeps liquid. The boiler is connected to the liquid reservoir through a feed passage and heats the liquid from the liquid reservoir to generate steam. The nozzle is connected to the boiler through a steam passage and directs the steam from the boiler to the outside. The discharge electrodes are located between the boiler and an opening of the nozzle. The intermediate electrode includes metal with antibacterial action and is located between the discharge electrodes. The intermediate electrode scatters metal ions derived from the metal by electric discharge generated between the intermediate electrode and one of the discharge electrodes as well as electric discharge generated between the intermediate electrode and the other of the discharge electrodes. In addition, components of a substance for separation of the metal ions are supplied between the boiler and the opening of the nozzle and the components prevent combination among the metal ions.

In this configuration, the metal ions become cores for the steam and the steam components condense on the cores to become minute. As a result, it is possible to provide human skin with steam (metal ions) having more suitable antibacterial action than minus ions generated with corona discharge (high voltage), self-induced radiation (tourmaline), electrostatic spray or water pulverization. Moreover, since the components of the substance for separation of the metal ions prevent combination among the metal ions, the antibacterial action derived from the metal ions can be give to the skin more uniformly.

In an embodiment of any of the first to third appliances, the metal with antibacterial action is platinum. In this case, effects (antibacterial action, anti-oxidative action, etc.) derived from the platinum can be provided to the skin.

In an embodiment of any of the first to third appliances, the substance is fluorine grease. In this case, components of the fluorine grease can prevent combination among the metal ions in a wide temperature range.

In an embodiment of any of the first to third appliances, the substance is applied to the inner surface of the steam passage. In this case, it is possible to increase chances that steam components and metal ions meet components of the substance.

In an embodiment of any of the first to third appliances, the skin care appliance further comprises a compound reservoir where the substance is kept. In this case, since quantity of the substance kept in the compound reservoir can be set in consideration of useful life of the skin care appliances, bother of replenishing the substance can be omitted.

In an embodiment of any of the first to third appliances, each of the discharge electrodes is formed with the compound reservoir. In this case, components of the substance for separation of the metal ions are applied to the discharge electrodes together with metal ions scattered from the electrodes, and therefore combination among the metal ions can be prevented suitably.

In an embodiment of any of the second and third appliances, the intermediate electrode is formed with the compound reservoir. Also in this case, combination among the metal ions can be prevented suitably.

In an embodiment of any of the first to third appliances, the skin care appliance further comprises a pair of electrode holders that support the pair of discharge electrodes, respectively. In addition, each of the electrode holders is formed with the compound reservoir. Also in this case, combination among the metal ions can be prevented suitably.

Preferably, the pair of discharge electrodes can be attached to or removed from the pair of electrode holders, respectively. In this case, since the discharge electrodes can be exchanged, each size of the electrodes need not be enlarged in consideration of useful life of the skin care appliances. Accordingly, the cost can be reduced.

### BRIEF DESCRIPTION OF THE DRAWINGS

Preferred embodiments of the invention will now be described in further details. Other features and advantages of the present invention will become better understood with regard to the following detailed description and accompanying drawings where:
FIG. 1 is a sectional view of a skin care appliance, in accordance with an embodiment of the present invention;
FIG. 2 is a perspective view of the appliance;
FIGs. 3A-3E are a left side view, a back view, a front view, a top view and a bottom view of the appliance, respectively;
FIG. 4 is a sectional view of an electric discharger in the appliance;
FIG. 5 is a sectional view of an electric discharger in a modified embodiment;
FIG. 6A is a sectional view of a discharge electrode in a varied embodiment and FIG. 6B shows the end-face of the electrode;
FIG. 7A is a sectional view of a discharge electrode in another varied embodiment and FIG. 7B shows the end-face of the electrode;
FIG. 8 is a sectional view of an intermediate electrode and a pipe prop in an alternate embodiment;
FIG. 9 is a sectional view of an intermediate electrode and a pipe prop in a varied embodiment; and
FIG. 10 is a sectional view of an intermediate electrode and a pipe prop in another varied embodiment.

### BEST MODE FOR CARRYING OUT THE INVENTION

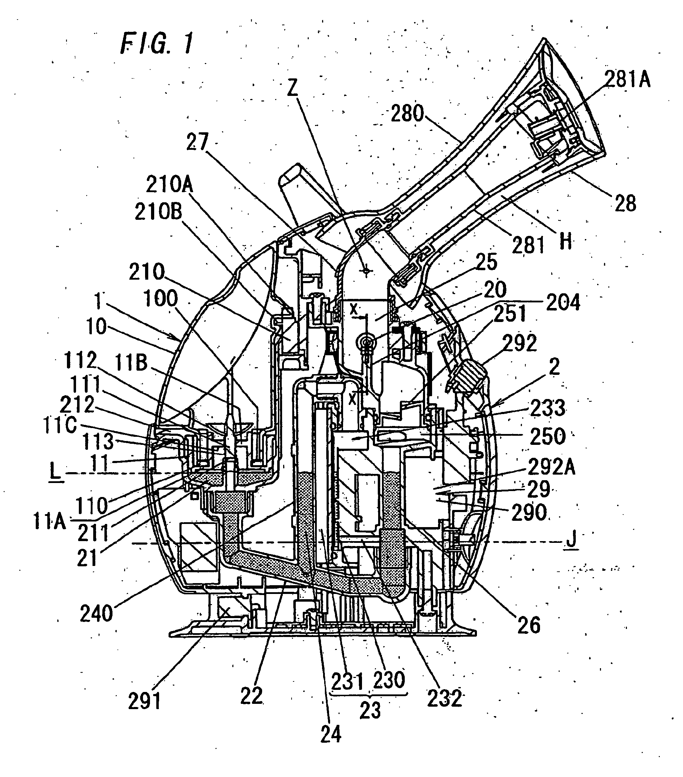

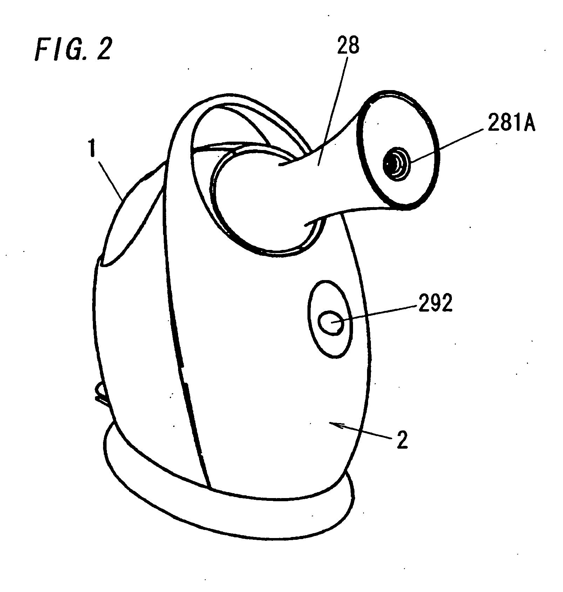

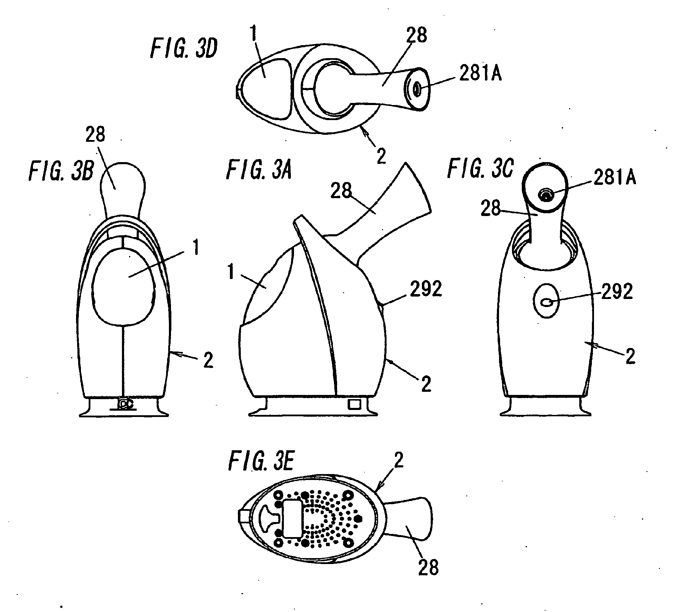

FIGs. 1, 2 and 3A-3E show an embodiment according to the present invention. This embodiment is a skin care appliance for steam beauty treatment (e.g., steam facial) with antibacterial action. For example, this appliance comprises a tank 1 and an appliance body 2 that incorporates a push-push type latch 210, a liquid reservoir 21, a feed passage 22, a boiler 23, a sub-feed passage 24, a steam passage 25, a condensate passage 26, a flexible duct (joint) 27, a nozzle 28, a controller 29 and an electric discharger 20.

The tank 1 can be attached to or detached from the appliance body 2 through the latch 210, and is formed of a tank body 10 and a cap 11 that is used to open and close an opening 100 of the tank body 10. In case that the tank 1 is attached to the appliance body 2, the tank body 10 is pushed by a user to press a button 210A of the latch 210, so that the tank 1 is fixed by a hook 210B projecting from the latch 210. In case that the tank 1 is detached from the appliance body 2, the tank body 10 is pushed by the user to press the button 210A, so that the hook 210B withdraws into the latch 210. Afterwards, the user can remove the tank 1 from the appliance body 2.

The cap 11 has a double cylindrical structure with a peripheral bottom 11A forming an inner cavity. This cap 11 is also provided with a ring-shaped packing 110, a stop pin 111, a dome-shaped packing 112, and a spring 113. The packing 110 is located between the bottom 11A and the edge of the opening 100. The pin 111 is inserted through a hole of the cap 11 and is supported between a pair of arms 11B and 11C projected from the inner face of the cap 11. The arms 11B and 11C support the pin 111 so that it can move vertically. The packing 112 has a hole through which the stop pin 111 is inserted, and is used to open and close the hole of the cap 11. The spring 113 is located at the outside of the arms 11B and 11C, and the stop pin 111 is inserted into the spring 113 to receive outward force. This stop pin 111 is pushed into the tank 1 by a projection pin 211 in the liquid reservoir 21 when the tank 1 has been attached to the appliance body 2. As a result, since the packing 112 opens the hole of the cap 11, the water in the tank 1 can flow in the liquid reservoir 21 through the hole. When the tank 1 is removed from the appliance body 2, the stop pin 111 projects from the tank 1 by the spring 113 and accordingly the packing 112 closes the hole of the cap 11. Therefore, in the embodiment, the tank 1 can be easily attached to or detached from the appliance body 2, and water leak from the tank 1 can be prevented. In addition, the tank 1 is detachable and water is supplied to the tank 1 with the downside (i.e., opening 100 side) up and accordingly excessive water supply with respect to the appliance body 2 can be prevented.

The liquid reservoir 21 is located below the cap 11 of the tank 1 attached to the appliance body 2, and keeps the water supplied from the tank 1. The bottom of this reservoir 21 is formed with teardrop shaped flow holes (not shown), and also the reservoir 21 is opened to air through, for example, a venthole (not shown). The feed passage 22 is, for example, feed water pipe located below the liquid reservoir 21 and the boiler 23, and can feed the water kept in the reservoir 21 to the boiler 23 side. The junction of the reservoir 21 and the passage 22 is sealed, and the junction of the passage 22 and the boiler 23 is also sealed. By locating the passage 22 below the boiler 23, reverse flow from the boiler 23 can be prevented.

The boiler 23 is connected to the liquid reservoir 21 through the feed passage 22 and heats the water from the reservoir 21 to generate steam. For example, this boiler 23 is formed of a boiling chamber 230, a heater 231, an inflow hole 232 and a steam exit 233. The boiling chamber 230 is located in contact with the heat side of the heater 231 and keeps boiling water derived from the water of the reservoir 21. The heater 231 is activated by the controller 29 to boil the water in the boiling chamber 230. The inflow hole 232 is located at the lower end of the boiling chamber 230 and the water from the feed passage 22 can flow in the boiling chamber 230 through the hole 232. In order to stabilize the pressure in the boiling chamber 230 and generate steam in a short time, the maximum inside dimension of the inflow hole 232 is shortened compared with the length of the inflow hole 232. For example, the diameter of the inflow hole 232 is set to 2.5 mm and the length of the inflow hole 232 is set to 18.0 mm. Thus, by regulating feed water from the feed passage 22 to the boiling chamber 230, water supply speed can be adjusted and a quantity of water suitable for the output (W) of the heater 231 can be heated. The steam exit 233 is located at the upper end of the boiling chamber 230 and steam generated in the boiling chamber 230 can flow thereof.

The sub-feed passage 24 is connected between an intermediate part of the feed passage 22 and the steam exit 233 of the boiler 23, and is located so that almost whole of the passage 24 is fixed in contact with the radiation side of the heater 231 with a fix member 240. The water temperature in the passage 24 is set to, for example, 90-98°C. In order to optimize a mixture ratio of water temperature, the cross section of the passage 24 is set to be the same as or larger than that of the passage 22. The junction of the passages 22 and 24 is sealed, or both are unified. It is desirable that this junction is located close to the boiling chamber 230 not to lower water temperature between the junction and the chamber 230. In addition, the junction of the passage 24 and the exit 233 is sealed to equilibrate the pressure in the passage 24 and the pressure in the exit 233. Therefore, the sub-feed passage 24 functions as a sub-heat mechanism and thereby pressure variation caused by steam condensation or the like can be reduced relatively and the pressures can be easily equilibrated. As an example, the sub-feed passage 24 may be fixed in contact with the heat side of the heater 231. In this case, the heater 231 needs to be enlarged so that the boiling chamber 230 and the sub-feed passage 24 can be fixed in contact with the heat side of the heater 231. As another example, the passage 24 may be separated from the passage 22 at the lower end of the passage 24 so that the water temperature in the reservoir 21 is not increased.

The steam passage 25 is connected to the steam exit 233 and the condensate passage 26 through, for example, a joint 250 in which cross passages are formed. Concretely, the passage 25 is connected to the passage 26 through upper and lower passages in the joint 250, and connected to the exit 233 through upper, left and right passages in the joint 250. Their junctions are also sealed. In addition, a bumping protective barrier 251 with uneven walls is formed in the passage 25 to prevent and protect bumping from the boiling chamber 230 when the heater 231 is operated. The protective barrier 251 can restrain sudden pressure variation and accordingly bumping can be prevented.

The condensate passage 26 is further connected to the lower end of the feed passage 22 to return condensate generated in the steam passage 25 and so on to the passage 22 in proximity to the inflow hole 232. Accordingly, steam output efficiency that is a rate of whole steam output to water supply can be improved. In the embodiment, the water in the liquid reservoir 21 is fed to the sub-feed passage 24 and the condensate passage 26 through the feed passage 22, and is fed to the boiling chamber 230 through the passage 22 and the inflow hole 232. And each surface of water in the passages 24 and 26 and the chamber 230 balances with the surface of the water in the liquid reservoir 21 as shown in the line "L" of FIG. 1. When the water in the appliance body 2 falls to the line "Y" of FIG. 1 during use, steam is no longer generated. After this use, remaining water in the appliance body 2 can be drained from an outlet 212.

The nozzle 28 has a double cylindrical structure with an outer cylinder 280 and an inner cylinder 281 of which tips are combined each other and between which an air layer H intervenes. In order to improve the insulation efficiency, the thickness of the air layer is set to, for example, 5 mm. However, not limited to this, heat insulation material (e.g., foam rubber) may be filled between the cylinders 280 and 281, or heat insulation material and air layer may be located between the cylinders 280 and 281. The inner cylinder 281 with an opening 281A is connected to the steam passage 25 through the flexible duct 27, and junctions of both ends of the duct 27 are sealed. Therefore, the nozzle 28 is movable about a pivot Z in the duct 27, and a user can adjust the angle of the opening 281A to a desired angle. Since the nozzle 28 projects from the appliance body 2, the opening 281A can be moved near to skin of a user. Accordingly, the user can direct steam at desired skin at ease.

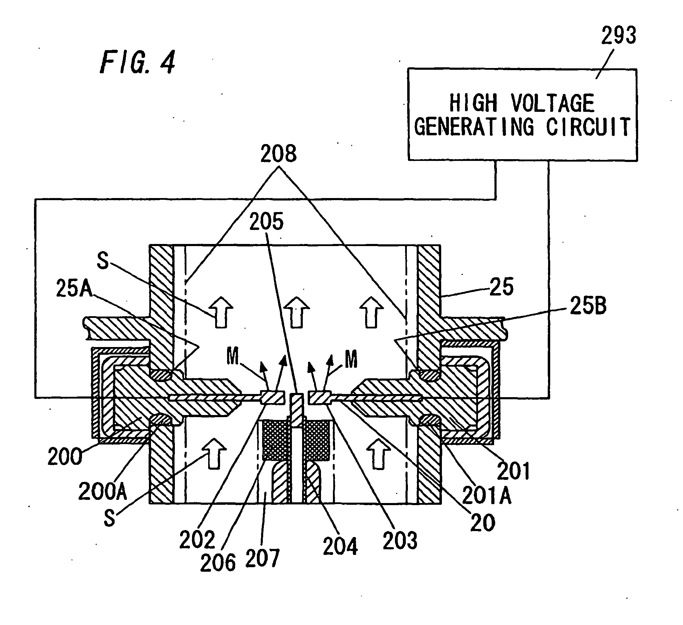

As shown in FIGs. 1 and 4, the controller 29 is formed of a power connector 291 connected with a power cord (not shown), a power button 292, various sensors (not shown), a high voltage generating circuit 293, a control circuit 290 (e.g., microcomputer, etc.) and so on. For example, when the button 292 is pressed and then a switch 292A is turned on, the controller 29 activates the heater 231 and the electric discharger 20 (circuit 293) in accordance with programs and detection signals from the sensors.

The electric discharger 20 includes electrode holders 200 and 201, pin-shaped discharge electrodes 202 and 203, a pipe prop 204 and a pin-shaped intermediate electrode 205, and is located in the vertical steam passage 25. This passage 25 is formed with horizontal facing holes 25A and 25B, in which the holders 200 and 201 are fit through O-rings 200A and 200B, respectively.

The electrodes 202 and 203 are respectively fixed by the holders 200 and 201, and are electrically connected to the high voltage generating circuit 293. As an example, the electrodes 202 and 203 can be attached to or detached from the holders 200 and 201, respectively. In this case, since the electrodes 202 and 203 can be exchanged, each size of the electrodes need not be enlarged in consideration of useful life of the skin care appliances. Accordingly, the cost can be reduced. Also, electrodes made of different metals can be attached. The circuit 293 is activated by the controller 29, and applies high voltage (e.g., DC voltage) across the electrodes 202 and 203 to generate electric discharge (e.g., arc discharge).

The prop 204 is made of, for example, SUS304 and is stood on the inside of bent part of the passage 25. The prop 204 is also located at a position where it does not receive the discharge between the electrodes 202 and 203. The electrode 205 is pressed into the hole of the prop 204 so as to position between the electrodes 202 and 203. That is, the electrode 205 can be attached to or detached from the tip of the prop 204. Accordingly, since the electrode 205 can be exchanged, size of the electrode need not be enlarged in consideration of useful life of the skin care appliances. However, not limited to this, the electrode 205 may be fixed to the tip of the prop 204 by a screw or fastening. In addition, the electrode 205 is not provided with voltage. In this case, since breakdown voltage necessary for discharge falls by intervention of the electrode 205, the voltage to be applied across the electrodes 202 and 203 can be decreased. As an example, DC high voltage may be applied across the electrodes 202 and 203 and the electrode 205. For example, the electrodes 202 and 203 is set to positive and the electrode 205 is set to negative. As another example, AC high voltage can be used instead of the DC high voltage. The discharge may be corona discharge, glow discharge or surface creepage.

According to an aspect of the present invention, the discharge electrodes and/or the intermediate electrode include metal with antibacterial action. In the embodiment, for example, the discharge electrodes 202 and 203 and the intermediate electrode 205 include metal (e.g., platinum) with antibacterial action. However, not limited this, the discharge electrodes and/or the intermediate electrode of the present invention may include rhodium, palladium or ruthenium. Among these, platinum has maximum anti-oxidative action. Accordingly, as shown in FIG. 4, the electrodes 202 and 205 scatter metal ions (metal minute particles M) derived from metal included in each of the electrodes 202 and 205 by discharge generated between the electrodes 202 and 205. Similarly, the electrodes 203 and 205 scatter metal ions (metal minute particles) derived from metal included in each of the electrodes 203 and 205 by discharge generated between the electrodes 203 and 205. That is, the electrodes 202, 203 and 205 constitute a metal minute particle generation means. In this case, the generated quantity of metal ions a unit time can be increased.

In another aspect of the present invention, components of a substance for separation of the metal ions are supplied between the boiler and the opening of the nozzle, and the components prevent combination among the metal ions. It is desirable that the substance for separation of the metal ions includes water-soluble compound. In the embodiment, the substance for separation of the metal ions is, for example, fluorine grease, and an impregnated member 206 containing the fluorine grease is fixed by pressing the tip of the prop 204 into a hole of the member 206. In addition, as shown in "207" and "208" of FIG. 4, the fluorine grease is applied to the prop 204 and the inner surface of the steam passage 24. As an example, the impregnated member 206 is non-woven fabric into which the fluorine grease is impregnated, and is located apart from the electrodes 202 and 203 by a minimum distance. That is, the impregnated member 206 is located as near as possible to the electrodes 202 and 203 so that leak does not occur between the member 206 and the electrodes 202 and 203 even in case that dew forms on the member 206 and the electrodes 202 and 203. As another example, the impregnated member 206 may be located on each of the electrodes 202 and 203 instead of the prop 204, or may be located on each of the electrodes 202 and 203 and the prop 204. In short, not limited to various embodiments, a substance for separation of the metal ions of the present invention can be put between the boiler and the opening of the nozzle, and preferably put in a range where the discharge between the discharge electrodes is not disturbed.

The operation of the present embodiment is now explained. When the button 292 is pressed and then a switch 292A is turned on, the controller 29 activates the heater 231 and the electric discharger 20 (circuit 293). The water in the boiling chamber 230 is heated by the heater 231 to flow in the steam passage 25 through the steam exit 233. In this process, when the water in the liquid reservoir 21 falls below the line of "L" in FIG. 1, air flows in the tank 1 and then the water in the tank 1 is supplied to the reservoir 21. The water is accelerated by gravitation to flow in the boiling chamber 230. In this case, while the water reaches the boiling chamber 230, the water from the reservoir 21 is mixed with the water in the sub-feed passage 24 and thereby the temperature of the water rises. Accordingly, the temperature of the boiling chamber 230 and the water therein can be prevented from falling, and generating time of steam can be shortened. In addition, since the water in the tank 1 is accelerated by gravitation to flow in the boiling chamber 230, it is possible to prevent the water level in the reservoir 21 from having large difference with respect to each water level in the passages 24 and 26 and the boiling chamber 230. As a result, since a large quantity of water supply in response to the large difference can be prevented, pulsation of steam can be restrained.

As shown in FIG. 4, the steam S from the steam exit 233 is discharged from the opening 281A of the nozzle 28 together with metal ions M scattered from the electric discharger 20. At this point, the metal ions become cores for the steam and the steam components condense on the cores to become minute. The steam components permeate into skin of a user to have water retention effect on the skin, and the metal ions have antibacterial action on the skin. In case that the metal with antibacterial action is platinum, coenzyme effect can be provided to the skin and also protective effect on the skin cells can be obtained by anti-oxidative action.

In addition, components of the substance for separation of the metal ions prevent combination among the metal ions. Concretely, in the steam passage 25, the grease melts through the temperature and moisture of the steam, and fluorine particles are mixed with the steam to stick the metal ions scattered from the discharger 20. As a result, combination among the metal ions is restrained. Since the various effects of the metal ions (metal minute particles) have a relationship of approximate inverse proportion to the sizes, drop of the various effects (e.g., coenzyme effect, etc.) can be restrained by preventing combination among the metal ions through the substance for separation of the metal ions.

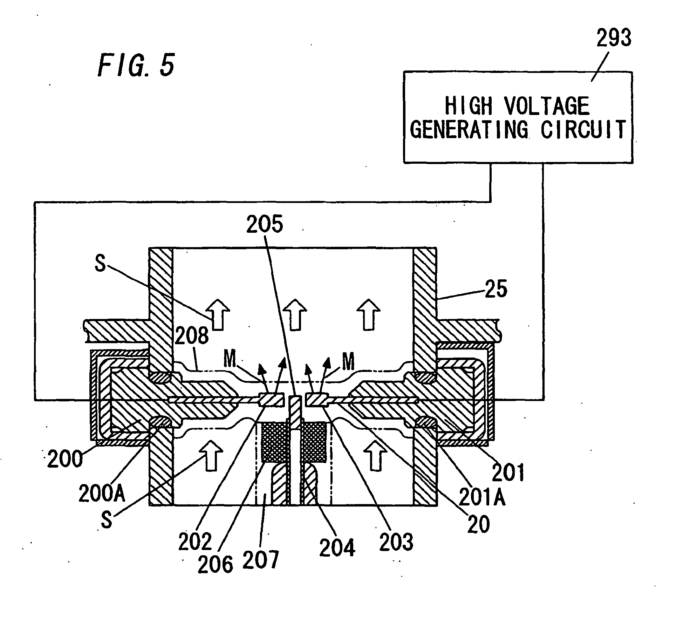

In a modified embodiment, fluorine grease is applied to a discharge part of the electric discharger 20 in stead of the inner surface of the steam passage 25. For example, as shown in "208" of FIG. 5, fluorine grease may be applied to the holders 200 and 201 as well as the electrodes 202 and 203, or may be applied to one set of holder and electrode.

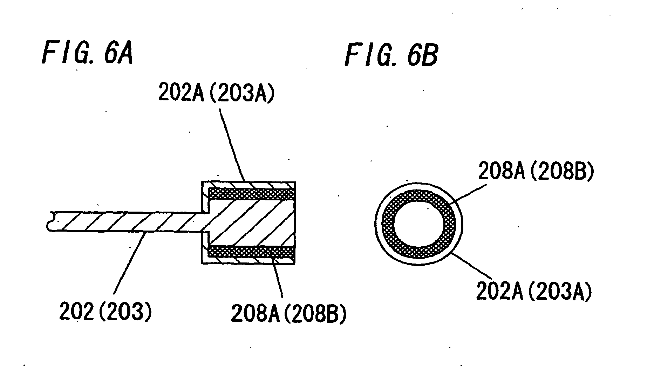

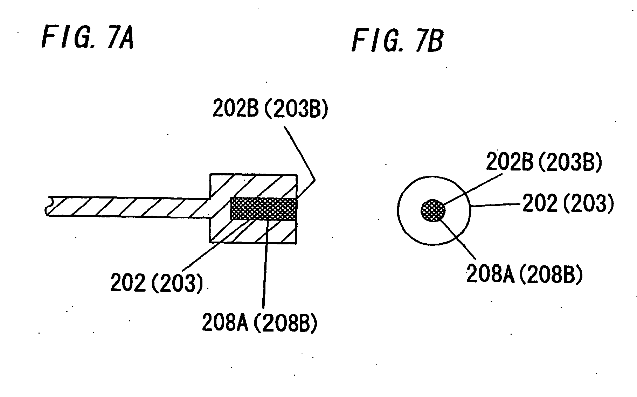

In a varied embodiment, as shown in FIGs. 6A and 6B, compound reservoirs 202A and 203A forming ring shaped grooves are located at the tips of the electrodes 202 and 203, respectively. In addition, a substance for separation of the metal ions (208A) is filled in each of the reservoir 202A and also a substance for separation of the metal ions (208B) is filled in each of the reservoir 203A. In this case, components of the substance for separation of the metal ions are applied to the discharge part of the electric discharger 20 together with metal ions (metal minute particles) scattered from the electrodes 202 and 203, and therefore combination among the metal ions can be prevented suitably. In another varied embodiment, as shown in FIGs. 7A and 7B, center grooves 202B and 203B as compound reservoirs are formed at the tips of the electrodes 202 and 203, in which substances for separation of the metal ions (208A and 208B) are filled, respectively.

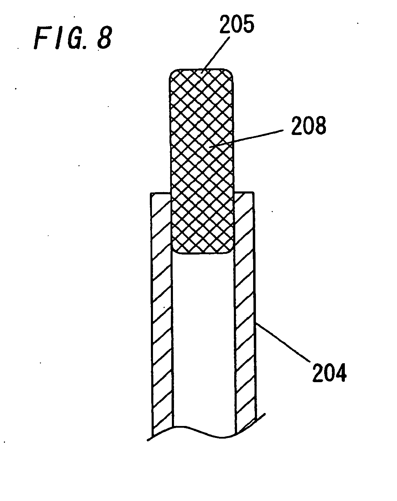

In an alternate embodiment, as shown in FIG. 8, the intermediate electrode 205 is a porous electrode, and a substance for separation of the metal ions (208) is applied to many small holes of the electrode. For example, the electrode 205 is a pin shaped porous platinum (low density platinum). In this case, components of the substance for separation of the metal ions are applied to the discharge part of the electric discharger 20 together with metal ions (metal minute particles) scattered from the electrodes 205, and therefore combination among the metal ions can be prevented suitably. The pipe prop 204 may also include a porous pipe (low density pipe) with a substance for separation of the metal ions applied to many small holes of the pipe.

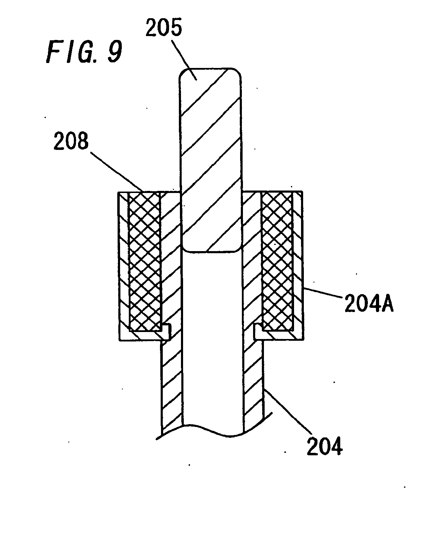

In a varied embodiment, as shown in FIG. 9, a compound reservoir 204A forming a ring shaped groove is located around the tip of the pipe prop 204 and a substance for separation of the metal ions (208) is filled in the reservoir 204A. Also in this case, combination among the metal ions can be prevented suitably.

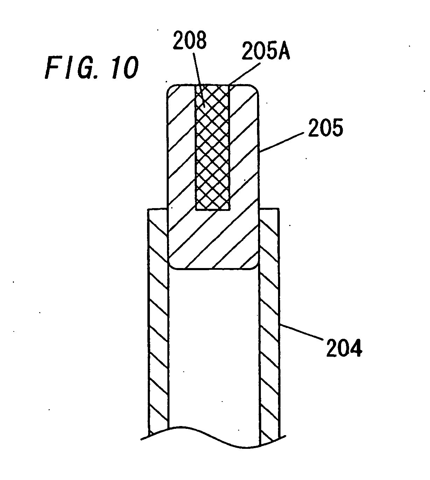

In another varied embodiment, as shown in FIG. 10, center groove 205A as a compound reservoir is formed at the tips of the intermediate electrode 205, in which a substance for separation of the metal ions (208) is filled. Also in this case, combination among the metal ions can be prevented suitably.

Although the present invention has been described with reference to certain preferred embodiments, numerous modifications and variations can be made by those skilled in the art without departing from the true spirit and scope of this invention as defined in the claims.

## Claims

1. A skin care appliance, comprising
a liquid reservoir (21) where liquid is kept;
a boiler (23) that is connected to the liquid reservoir (21) through a feed passage (22) and heats the liquid from the liquid reservoir (21) to generate steam;
a nozzle (28) that is connected to the boiler (23) through a steam passage (25) and directs the steam from the boiler (23) to the outside; and
a pair of discharge electrodes (202, 203) that include metal with antibacterial action and are located between the boiler (23) and an opening of the nozzle (28), said electrodes (202, 203) scattering metal ions derived from the metal by electric discharge generated between the electrodes (202, 203);
**characterized in that** components of a substance for separation of the metal ions are supplied between the boiler (23) and the opening of the nozzle (28), the components preventing combination among the metal ions.

2. The skin care appliance of claim 1, wherein the metal is platinum.

3. The skin care appliance of claim 1, wherein the substance is fluorine grease (207, 208, 208A, 208B).

4. The skin care appliance of claim 1, wherein the substance is applied to the inner surface of the steam passage (25).

5. The skin care appliance of claim 1, further comprising a compound reservoir (202A, 203A, 202B, 203B, 204A) where the substance is kept.

6. The skin care appliance of claim 5, wherein each of the discharge electrodes (202, 203) is formed with the compound reservoir (202A, 203A, 202B, 203B).

7. The skin care appliance of claim 5, further comprising a pair of electrode holders (200, 201) that support the pair of discharge electrodes (202, 203), respectively wherein each of the electrode holders (200, 201) is formed with the compound reservoir.

8. The skin care appliance of claim 7 wherein the pair of discharge electrodes (202, 203) can be attached to or removed from the pair of electrode holders (200, 201), respectively.

9. A skin care appliance, comprising
a liquid reservoir (21) where liquid is kept;
a boiler (23) that is connected to the liquid reservoir (21) through a feed passage (22) and heats the liquid from the liquid reservoir (21) to generate steam;
a nozzle (28) that is connected to the boiler (23) through a steam passage (25) and directs the steam from the boiler (23) to the outside;
a pair of discharge electrodes (202, 203) located between the boiler (23) and an opening of the nozzle (28); and
an intermediate electrode (205) that includes metal with antibacterial action and is located between the discharge electrodes (202, 203), said intermediate electrode (205) scattering metal ions derived from the metal by electric discharge generated between the intermediate electrode (205) and one of the discharge electrodes (202, 203) as well as electric discharge generated between the intermediate electrode (205) and the other of the discharge electrodes (202, 203);
**characterized in that** components of a substance for separation of the metal ions are supplied between the boiler (23) and the opening of the nozzle (28), the components preventing combination among the metal ions.

10. The skin care appliance of claim 9, wherein the metal is platinum.

11. The skin care appliance of claim 9, wherein the substance is fluorine grease (207, 208, 208A, 208B).

12. The skin care appliance of claim 9, wherein the substance is applied to the inner surface of the steam passage (25).

13. The skin care appliance of claim 9, further comprising a compound reservoir (202A, 203A, 202B, 203B, 204A, 205A) where the substance is kept.

14. The skin care appliance of claim 13, wherein each of the discharge electrodes (202, 203) is formed with the compound reservoir (202A, 203A, 202B, 203B).

15. The skin care appliance of claim 13, wherein the intermediate electrode (205) is formed with the compound reservoir (205A).

16. The skin care appliance of claim 13, further comprising a pair of electrode holders (200, 201) that support the pair of discharge electrodes (202, 203), respectively wherein each of the electrode holders (200, 201) is formed with the compound reservoir.

17. The skin care appliance of claim 16, wherein the pair of discharge electrodes (202, 203) can be attached to or removed from the pair of electrode holders (200, 201), respectively.

## Patentansprüche

1. Hautpflegegerät, das umfasst:
einen Flüssigkeitsbehälter (21), in dem Flüssigkeit aufbewahrt wird,
einen Boiler (23), der über einen Zufuhrdurchlass (22) mit dem Flüssigkeitsbehälter (21) verbunden ist und die Flüssigkeit aus dem Flüssigkeitsbehälter (21) erhitzt, um Dampf zu erzeugen,
eine Düse (28), die über einen Dampfdurchlass (25) mit dem Boiler (23) verbunden ist und den Dampf vom Boiler (23) nach außen lenkt, und
ein Paar Entladungselektroden (202, 203), die Metall mit antibakterieller Wirkung aufweisen und zwischen dem Boiler (23) und einer Öffnung der Düse (28) angeordnet sind, wobei die Elektroden (202, 203) Metallionen streuen, die durch eine zwischen den Elektroden (202, 203) erzeugte elektrische Entladung aus dem Metall gewonnen werden,
**dadurch gekennzeichnet, dass** zwischen dem Boiler (23) und der Öffnung der Düse (28) Bestandteile einer Substanz zum Trennen der Metallionen zugeführt werden, wobei die Bestandteile eine Kombination unter den Metallionen verhindern.

2. Hautpflegegerät nach Anspruch 1, wobei das Metall Platin ist.

3. Hautpflegegerät nach Anspruch 1, wobei die Substanz ein Fluorschmiermittel (207, 208, 208A, 208B) ist.

4. Hautpflegegerät nach Anspruch 1, wobei die Substanz auf die Innenfläche des Dampfdurchlasses (25) aufgetragen wird.

5. Hautpflegegerät nach Anspruch 1, das ferner einen Zusammensetzungsbehälter (202A, 203A, 202B, 203B, 204A) umfasst, in dem die Substanz aufbewahrt wird.

6. Hautpflegegerät nach Anspruch 5, wobei die Entladungselektroden (202, 203) jeweils mit dem Zusammensetzungsbehälter (202A, 203A, 202B, 203B) ausgebildet sind.

7. Hautpflegegerät nach Anspruch 5, das ferner ein Paar Elektrodenhalter (200, 201) umfasst, die das Paar Entladungselektroden (202, 203) tragen, wobei die Elektrodenhalter (200, 201) jeweils mit dem Zusammensetzungsbehälter ausgebildet sind.

8. Hautpflegegerät nach Anspruch 7, wobei das Paar Entladungselektroden (202, 203) an dem Paar Elektrodenhalter (200, 201) befestigt beziehungsweise davon entfernt werden kann.

9. Hautpflegegerät, das umfasst:
einen Flüssigkeitsbehälter (21), in dem Flüssigkeit aufbewahrt wird,
einen Boiler (23), der über einen Zufuhrdurchlass (22) mit dem Flüssigkeitsbehälter (21) verbunden ist und die Flüssigkeit aus dem Flüssigkeitsbehälter (21) erhitzt, um Dampf zu erzeugen,
eine Düse (28), die über einen Dampfdurchlass (25) mit dem Boiler (23) verbunden ist und den Dampf vom Boiler (23) nach außen lenkt,
ein Paar Entladungselektroden (202, 203), die zwischen dem Boiler (23) und einer Öffnung der Düse (28) angeordnet sind, und
eine Zwischenelektrode 205, die Metall mit antibakterieller Wirkung aufweist und zwischen den Entladungselektroden (202, 203) angeordnet ist, wobei die Zwischenelektrode (205) Metallionen streut, die durch eine elektrische Entladung aus dem Metall gewonnen werden, die zwischen der Zwischenelektrode (205) und einer der Entladungselektroden (202, 203) erzeugt wird, sowie aus einer elektrischen Entladung, die zwischen der Zwischenelektrode (205) und der jeweils anderen Entladungselektrode (202, 203) erzeugt wird,
**dadurch gekennzeichnet, dass** zwischen dem Boiler (23) und der Öffnung der Düse (28) Bestandteile einer Substanz zum Trennen der Metallionen zugeführt werden, wobei die Bestandteile eine Kombination unter den Metallionen verhindern.

10. Hautpflegegerät nach Anspruch 9, wobei das Metall Platin ist.

11. Hautpflegegerät nach Anspruch 9, wobei die Substanz ein Fluorschmiermittel (207, 208, 208A, 208B) ist.

12. Hautpflegegerät nach Anspruch 9, wobei die Substanz auf die Innenfläche des Dampfdurchlasses (25) aufgetragen wird.

13. Hautpflegegerät nach Anspruch 9, ferner einen Zusammensetzungsbehälter (202A, 203A, 202B, 203B, 204A, 205A) umfassend, in dem die Substanz aufbewahrt wird.

14. Hautpflegegerät nach Anspruch 13, wobei die Entladungselektroden (202, 203) jeweils mit dem Zusammensetzungsbehälter (202A, 203A, 202B, 203B) ausgebildet sind.

15. Hautpflegegerät nach Anspruch 13, wobei die Zwischenelektrode (205) mit dem Zusammensetzungsbehälter (205A) gebildet ist.

16. Hautpflegegerät nach Anspruch 13, ferner ein Paar Elektrodenhalter (200, 201) umfassend, die das Paar Entladungselektroden (202, 203) tragen, wobei die Elektrodenhalter (200, 201) jeweils mit dem Zusammensetzungsbehälter ausgebildet ist.

17. Hautpflegegerät nach Anspruch 16, wobei das Paar Entladungselektroden (202, 203) an dem Paar Elektrodenhalter (200, 201) befestigt beziehungsweise davon entfernt werden kann.

## Revendications

1. Appareil de soin pour la peau, comportant :
un réservoir de liquide (21) dans lequel du liquide est conservé,
une chaudière (23) qui est reliée au réservoir de liquide (21) par un passage d'alimentation (22) et qui chauffe le liquide provenant du réservoir de liquide (21) afin de générer de la vapeur,
une buse (28) qui est reliée à la chaudière (23) par un passage de vapeur (25) et qui dirige la vapeur provenant de la chaudière (23) vers l'extérieur, et
une paire d'électrodes de décharge (202, 203) qui incluent un métal à action antibactérienne et qui sont situées entre la chaudière (23) et une ouverture de la buse (28), lesdites électrodes (202, 203) diffusant des ions métalliques issus du métal par une décharge électrique générée entre les électrodes (202, 203),
**caractérisé en ce que** des composants d'une substance pour la séparation des ions métalliques sont fournis entre la chaudière (23) et l'ouverture de la buse (28), les composants empêchant une combinaison entre les ions métalliques.

2. Appareil de soin pour la peau selon la revendication 1, dans lequel le métal est le platine.

3. Appareil de soin pour la peau selon la revendication 1, dans lequel la substance est une graisse fluorée (207, 208, 208A, 208B).

4. Appareil de soin pour la peau selon la revendication 1, dans lequel la substance est appliquée sur la surface intérieure du passage de vapeur (25).

5. Appareil de soin pour la peau selon la revendication 1, comportant en outre un réservoir de composé (202A, 203A, 202B, 203B, 204A) dans lequel la substance est conservée.

6. Appareil de soin pour la peau selon la revendication 5, dans lequel chacune des électrodes de décharge (202, 203) est formée avec le réservoir de composé (202A, 203A, 202B, 203B).

7. Appareil de soin pour la peau selon la revendication 5, comportant en outre une paire de porte-électrodes (200, 201) qui supportent la paire d'électrodes de décharge (202, 203), respectivement, dans lequel chacun des porte-électrodes (200, 201) est formé avec le réservoir de composé.

8. Appareil de soin pour la peau selon la revendication 7, dans lequel la paire d'électrodes de décharge (202, 203) peut être fixée sur la paire de porte-électrodes (200, 201) ou être retirée de celle-ci, respectivement.

9. Appareil de soin pour la peau, comportant :
un réservoir de liquide (21) dans lequel du liquide est conservé,
une chaudière (23) qui est reliée au réservoir de liquide (21) par un passage d'alimentation (22) et qui chauffe le liquide provenant du réservoir de liquide (21) afin de générer de la vapeur,
une buse (28) qui est reliée à la chaudière (23) par un passage de vapeur (25) et qui dirige la vapeur provenant de la chaudière (23) vers l'extérieur,
une paire d'électrodes de décharge (202, 203) situées entre la chaudière (23) et une ouverture de la buse (28), et
une électrode intermédiaire (205) qui inclut un métal à action antibactérienne et qui est située entre les électrodes de décharge (202, 203), ladite électrode intermédiaire (25) diffusant des ions métalliques issus du métal par une décharge électrique générée entre l'électrode intermédiaire (205) et l'une des électrodes de décharge (202, 203) ainsi qu'une décharge électrique générée entre l'électrode intermédiaire (205) et l'autre des électrodes de décharge (202, 203),
**caractérisé ce que** des composants d'une substance pour la séparation des ions métalliques sont fournis entre la chaudière (23) et l'ouverture de la buse (28), les composants empêchant une combinaison entre les ions métalliques.

10. Appareil de soin pour la peau selon la revendication 9, dans lequel le métal est le platine.

11. Appareil de soin pour la peau selon la revendication 9, dans lequel la substance est de la graisse fluorée (207, 208, 208A, 208B).

12. Appareil de soin pour la peau selon la revendication 9, dans lequel la substance est appliquée sur la surface intérieure du passage de vapeur (25).

13. Appareil de soin pour la peau selon la revendication 9, comportant en outre un réservoir de composé (202A, 203A, 202B, 203B, 204A, 205A) dans lequel la substance est conservée.

14. Appareil de soin pour la peau selon la revendication 13, dans lequel chacune des électrodes de décharge (202, 203) est formée avec le réservoir de composé (202A, 203A, 202B, 203B).

15. Appareil de soin pour la peau selon la revendication 13, dans lequel l'électrode intermédiaire (205) est formée avec le réservoir de composé (205A).

16. Appareil de soin pour la peau selon la revendication 13, comportant en outre une paire de porte-électrodes (200, 201) qui supportent la paire d'électrodes de décharge (202, 203), respectivement, dans lequel chacun des porte-électrodes (200,201) est formé avec le réservoir de composé.

17. Appareil de soin pour la peau selon la revendication 16, dans lequel la paire d'électrodes de décharge (202, 203) peut être fixée sur la paire de porte-électrodes (200, 201) ou être retirée de celle-ci, respectivement.
